# EUROPEAN PATENT APPLICATION

(11) **EP 3 494 902 A1**
(43) Date of publication of application: **12.06.2019**
(21) Application number: 17020560.3
(22) Date of filing: 07.12.2017
(51) Int. Cl.: A61B 17/12, A61B 17/00

(54) **IMPLANTABLE AND REPOSITIONABLE CARDIOVASCULAR DEVICE**

(71) Applicant: Peter Osypka Stiftung, 79639 Grenzach-Wyhlen (DE)
(72) Inventor: OSYPKA, Peter, 79639 Grenzach-Wyhlen (DE)

(57) **Abstract**

The invention relates to an implantable cardiovascular device comprising a body which is expandable from a collapsed structure to an expanded structure, the body has a rotation axis and comprises one or more anchor members being attached to the body surface ; the anchor member has an end portion acting as anchor element and extending outwardly from the surface of the body; characterized in that the angle between the longitudinal axis of the end portion of the anchor member and the rotation axis of the body is about 90 degree and that the angle between the longitudinal axis of the end portion of the anchor member and the surface of the body is between 0 and 45 degree, preferably 0 to 30 degree.

## Description

The invention relates to an implantable cardiovascular device such as an occluder having anchor members being arranged such that positioning and repositioning of the device can be effected before device anchoring. After positioning of the device is completed and the device fits perfectly device anchoring is done. Thus the act of positioning and repositioning is completely separated from the act of anchoring. The device enables easy positioning and repositioning avoiding any tissue damage.

### Background

Cardiovascular devices that are inserted into a body lumen to close the lumen such as for example a left atrial appendage (LAA) closure device normally have anchor members being operably coupled to the device, said members being configured to engage the device within the body lumen thus securing positioning and correct placement of the device.

When inserting such cardiovascular devices even experienced persons might have difficulties in guiding the cardiovascular device into the appropriate position. Often repositioning is required. Repositioning is a problem each time when the position of a device with anchor members must be corrected. In this case repositioning mostly results in tissue damage as the anchor members which have already pierced the surrounding tissue must be pulled out.

There is thus a need for a cardiovascular device that reduces the risk of damage of the surrounding tissue during positioning and repositioning.

### Solution:

The device comprises anchor members which do not pierce the tissue while inserting and repositioning the device due to their specific alignment and due to their flexibility. The device is inserted by means of a delivery catheter. The device is pushed out of the delivery catheter under turning thereby expanding. The alignment of the anchor member is such that as the device is turned in one direction the anchor members follow the rotation direction without piercing the tissue. When the correct position of the device is reached the device is shortly turned in the other direction and the anchor members anchor.

### Invention

The invention relates to an implantable cardiovascular device comprising a body which is expandable from a collapsed structure to an expanded structure, the body has a rotation axis and comprises one or more anchor members being attached to the body surface ; the anchor member has an end portion acting as anchor element and extending outwardly from the surface of the body; characterized in that the angle between the longitudinal axis of the end portion of the anchor member and the rotation axis of the body is about 90 degree and that the angle between the longitudinal axis of the end portion of the anchor member and the surface of the body is between 0 and 45 degree, preferably 0 to 30 degree.

In one embodiment the cardiovascular device is a closure device being implantable into a human body cavity such as into the left atrial appendage.

The body is made of any biocompatible material. Suitable are biocompatible metals, e.g. stainless steel, MP35N, gold, platinum, Pt/lr, Cu/Ni alloy like ISOTAN; or tungsten or any shape memory materials, preferably nitinol. The body may also be made of biocompatible plastic.

The body has for example an open structured surface. The surface is preferably a metallic surface, preferably self-expandable and made of nitinol. The body may e.g. have a tubular or cylindrical shape. The body is deformable. When inserting the body into a body lumen, such as the left atrial appendage the body may take the irregular shape of the left atrial appendage cavity.

The term "open structured surface" preferably refers to mesh structures formed of metallic multi wires diagonally crossing each other; but different alternatives such as scaffold structures, framework structures, struts, helical structures (coils, spirals) slotted tubes, cut tubes and the like are usable depending on its application.

The body is rotatable around the rotation axis. In the use position, when the cardiovascular device is inserted into e.g. the left atrial appendage two rotation directions have to be distinguished.
The first rotation direction is mostly a clockwise direction which is used when inserting the device. It may therefore be called "insertion direction". The second rotation direction, opposite to the first direction is accordingly a counterclockwise direction which is used to anchor the device. It may therefore be called "anchor direction". The rotation direction depends on the perspective of the observer. The clockwise direction is viewed from a proximal side, the side next to the person inserting the device.

There is no limitation in rotating the device in the "insertion direction", so that positioning and repositioning of the device is possible as long as the rotation direction is not changed.
The extent of rotation is limited in the opposite direction, in the "anchor direction" as the anchor elements grip the tissue.

The angle formed between the longitudinal axis of the end portion of the anchor member and the surface of the body is an angle between a tangent line drawn in the point of intersection between the surface body and the end portion of the anchor element.

The aim of the present invention is the kind of anchor member, the manner in which the anchor member is attached to the body and the manner in which the end portion of the anchor member is aligned. The alignment of the end portion of the anchor member is reached by pre-shaping the anchor member before using the device fulfilling the above mentioned requirements for the angle between the longitudinal axis of the end portion of the anchor member and the rotation axis and the surface of the body respectively.

The anchor member functions in a similar way as a thread. The end portion of the anchor member engages when, and only when turning the cardiovascular device in one single direction, in the so called "anchoring direction". The opposite direction, the so called "insertion direction" is the rotation direction during insertion and placement of the cardiovascular device. When the device is placed correctly the rotation direction is changed.

The anchor member consists preferably of a thin metallic wire having a thickness in the range of 0,05mm to 0.5mm, preferably 0.05mm to 0.3mm and a length in the range of 1mm to 3mm. Alternatively a metallic coil may be used.

At least one anchor member must be present. Preferably the device has more than one anchor member, e.g. 2 to 10 anchor members or 4 to 8 anchor members.
The anchor member is made of a biocompatible material such as a biocompatible metal, preferably nitinol or of biocompatible plastic.

The anchor member is preferably made of one single wire. The anchor member is attached to the surface of the cardiovascular device. Any common attaching techniques are conceivable.

When the metallic body is a mesh, the anchor member may be integrated within the mesh structure, preferably may be braided into the mesh. Alternatives are to weld or to knot the anchor member to the mesh structure preferably at a crossing point of the mesh.

The decisive portion of the anchor member is the end portion. This end portion is the actual anchor element. This end portion extends outwardly of the body of the cardiovascular device. This end portion is pre-shaped before inserting the cardiovascular device such that after the device is inserted and is in its expanded structure the end portion extends outwardly in a direction that is substantially perpendicular to rotational axis of the body of the cardiovascular device and the angle between the longitudinal axis of the end portion of the anchor member and the surface of the body is between 0 and 45 degree, preferably 0 to 30 degree.

The end portion of the anchor member may be shaped as any common fixing element such as a straight wire, a curved wire, a sinus shaped wire, a tine shaped wire, an U-shaped wire, a V-shaped wire, a loop, a sling, a hook, a double hook, a litz wire, a barb, a T-bar, a screw, and the like. In one embodiment the end portion of the anchor member is a straight wire. Important is not the form of the end portion but its alignment.

When inserting the cardiovascular device the anchor member first follows the so called "insertion direction" and does not act as anchor element. Due to its pre-shaped angle the anchor element follows the rotational movement and does not pierce or damage any tissue. The situation is completely different when changing the rotation direction. The anchor element anchors immediately after changing the rotation direction. A short tug in the so called "anchoring direction" and the anchor element grips the surrounding tissue.

The anchor member can be attached at any part of the mesh structure. In case of a plurality of anchor members, they might be attached such that the end portions are arranged circularly or diagonally. Any desired distribution of the anchor members over the mesh structure is possible. Thus an optimal anatomical adaption may be reached.

As outlined above, the cardiovascular device may be a closure device being implantable into a human body cavity such as the left atrial appendage. A left atrial closure device normally consists of a body part placed into the LAA cavity and a closure part, covering the entrance of the LAA. Anchoring members are attached to and distributed over the body part.

As the focus of the present invention is on the alignment of the anchor member, any type of LAA occluder is applicable. A suitable occluder may be one as described in the international publication WO2016087145 comprising an occluder formed according to the individual LAA anatomy.

The closure device is generally connected to an introducer, for example by threads. The thread supports the rotation when inserting the LAA occluder. The person inserting the device first turns the device in the so called "insertion direction", normally the clockwise direction (seen from the proximal perspective) until the device is deployed and placed correctly. The thread prevents an unintentional movement in the opposite direction. The introducer may also be a so called "catcher" a forceps catheter with stainless steel tentacles.

The accompanying drawings illustrate a preferred embodiment of the present invention. However the present invention is not to be construed as being limited to the drawings.

The numbering of the drawings is as follows.

| | |
|---|---|
| Rotation axis | 1 |
| Mesh structure | 2 |
| Anchor member | 3 |
| end portion of the anchor member | 4 |
| distal end of the braiding | 5 |
| proximal end of the braiding | 6 |
| longitudinal axis of the end portion | 7 |
| tangent | 8 |
| LAA cavity | 9 |
| Mesh body of an LAA occluder | 10 |
| Closure part of an LAA occluder | 11 |
| Tissue of the LAA cavity | 12 |

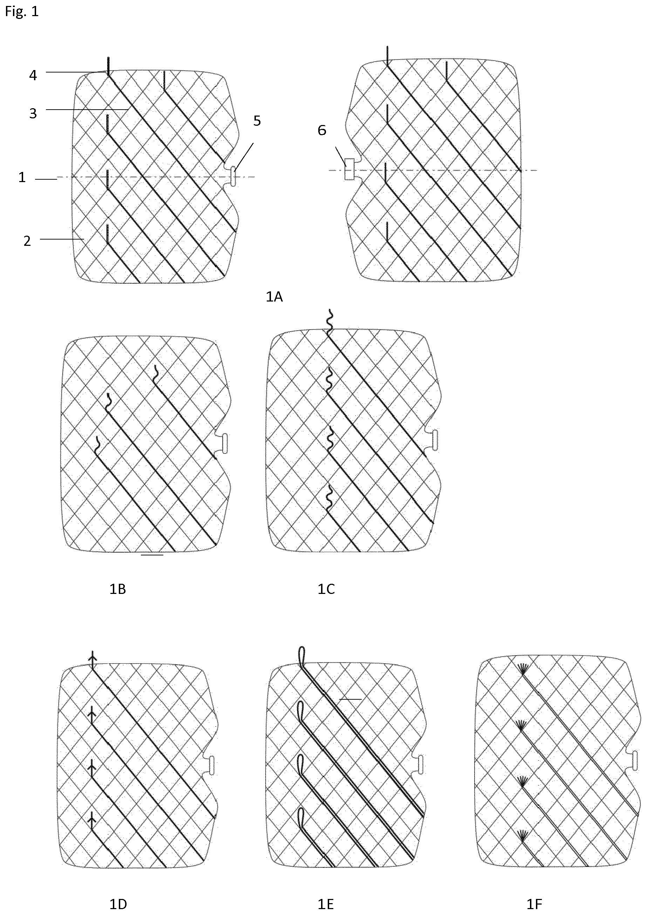

Fig. 1 to 4 show in a two dimensional side view several mesh structures used as occlusion device in an expanded configuration. The occlusion device includes a self-expanding metallic mesh structure (2) and a plurality of anchor members (3). The mesh structure is rotatable around the rotation axis (1). The mesh is braided. The anchor member (3) is a metallic wire attached to one of the mesh-wires having an end portion (4) which functions as anchor element.

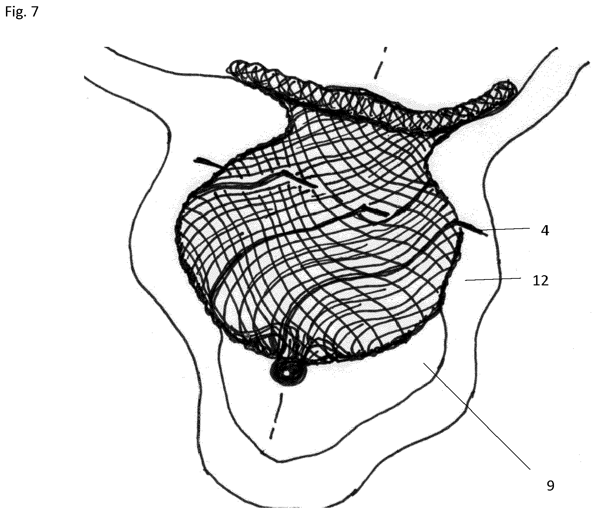

In the embodiment shown in Fig. 1 the anchor member (3) is a metallic wire integrated with the mesh structure (2), thus running along with one of the diagonal wires of the mesh. The end portion (4) of the metallic wire is a straight wire extending outwardly from the mesh perpendicular to the rotational axis (1) of the mesh structure.
In this two-dimensional view it is not possible to demonstrate the essential tangential alignment. This is demonstrated in Fig.7 showing the principle of the present invention.

The device shown in Fig. 1A is shown in two different versions, having a distal end part of the braiding (5) or a proximal end part of the braiding being part of a thread (6) acting as coupler to an introducer. If the braiding has a distal end part, a catcher is used to insert the device.

In the embodiment shown in 1B the end portion of the anchor member is curved.
In the embodiment shown in 1C the end portion of the anchor member is sinus shaped.
In the embodiment shown in 1D the end portion of the anchor member is barb shaped.
In the embodiment shown in 1E the end portion of the anchor member is a loop.
In the embodiment shown in 1F the end portion of the anchor member is a litz wire.

Each anchor member is aligned perpendicular to the rotational axis of the mesh structure.

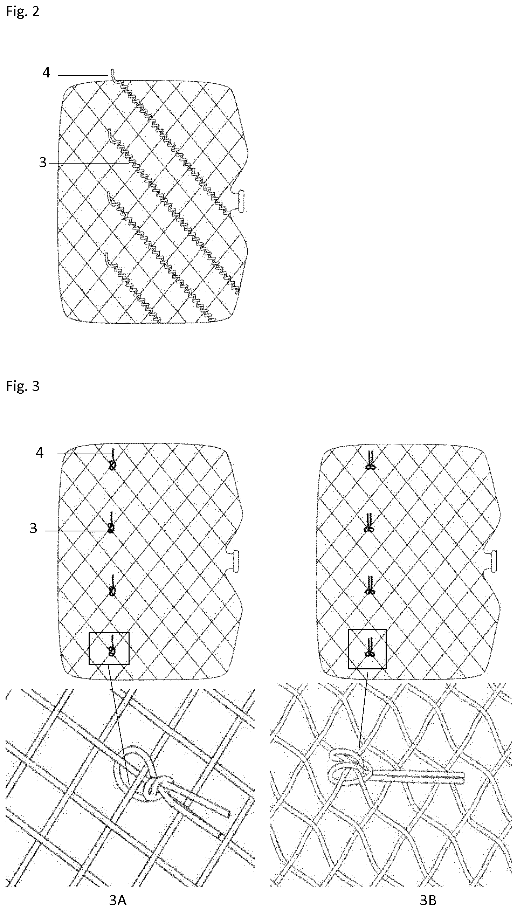

In the embodiments shown in Fig. 2 the anchor member (3) is a metallic coil being coiled around one of the diagonal wires of the mesh. The end portion (4) of the coil is bent outwards such that it extends perpendicular to the rotational axis of the mesh structure.

In the embodiments shown in Fig. 3 the anchor member (3) is a short wire being knotted to the mesh structure in a crossing point. The end portion of the knot (4) is bent outwards perpendicular to the rotational axis of the mesh structure.

In the embodiment shown in 3A the knot has one end wire forming the anchor element.
In the embodiment shown in Fig. 3B the knot has two end wires forming the anchor element.

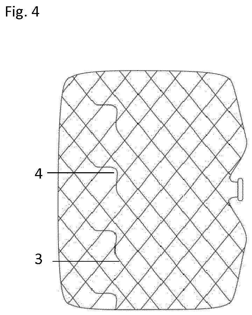

In the embodiment shown in Fig. 4 the wires of the mesh structure have been modified to form a protrusion said protrusion acting as anchor element being bent perpendicular to the rotation axis. The number of protrusions may vary as well as their distribution over the mesh structure.

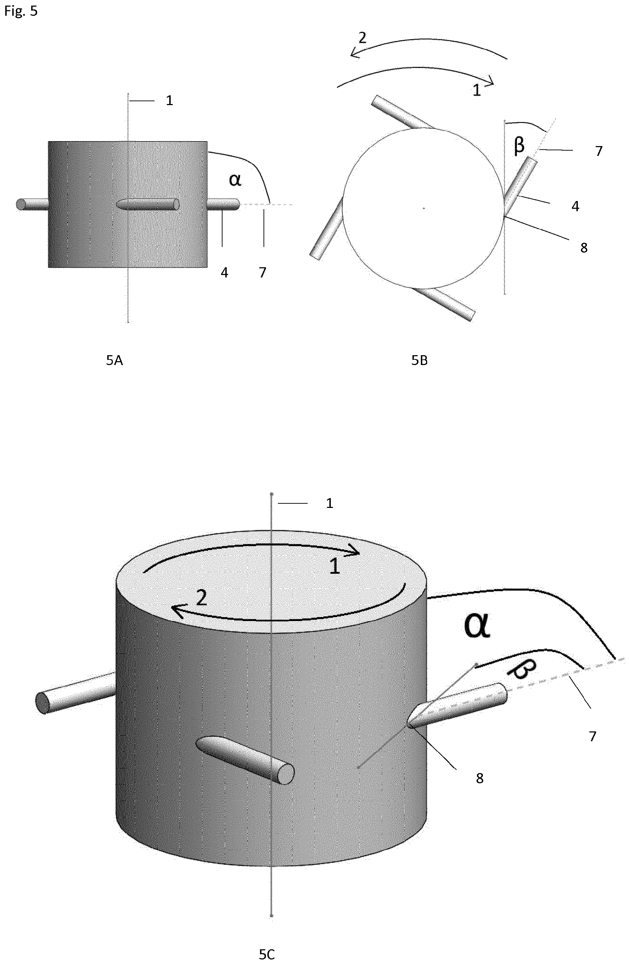

Fig. 5 shows schematically how the end portion of the anchor member must be aligned in a two-dimensional view but taking into account two different planes.

Fig. 5A is a two-dimensional side view showing the implantable cylindrical body from the side thus to be seen as a square. This view corresponds to the view shown in Fig. 1 and shows that the longitudinal axis (7) of the end portion (4) of the anchor member is perpendicular to the rotation axis (1). The angle α is 90 degree.

Fig. 5B is a two dimensional top view showing the implantable cylindrical body from the top thus to be seen as a circle. The perspective is a proximal view. The longitudinal axis (7) of the end portion (4) of the anchor member forms an angle β of about 30 degree to the body surface. This is shown drawing a tangent (8) in the point of intersection between the surface body and the end portion (4) of the anchor member.
The rotation direction is marked with an arrow. 1 = clockwise, 2 = counterclockwise.

Fig. 5C shows schematically how the anchor element must be aligned in a three-dimensional view. The reference numbers correspond to the numbers used in Fig. 5A and 5B.

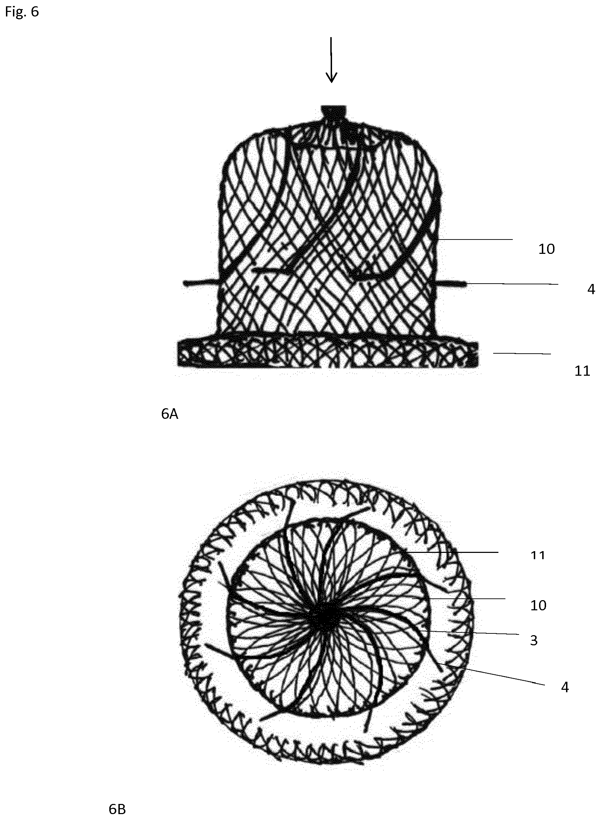

Fig. 6 shows the closure device used to close a left atrial appendage. The closure device consists of a body part (10) and a closure part (11). The device is braided of nitinol wires. The anchor members (3) are made of the same nitinol wire and are braided into the mesh. The end portion (4) of the metallic wire is a straight wire extending outwardly from the mesh perpendicular to the rotational axis of the mesh structure.

Fig. 6A is a side view of the closure device.
Fig. 6B is a top view showing the device from the distal perspective as marked by an arrow in fig. 6A

Fig. 7 shows the closure device inserted into the LAA cavity (9). The mesh is deformable and takes the irregular shape of the left atrial appendage cavity. The end portions (4) of the anchor member are fixed in the tissue (12).

## Claims

1. An implantable cardiovascular device comprising a body which is expandable from a collapsed structure to an expanded structure, the body has a rotation axis and comprises one or more anchor members being attached to the body surface ; the anchor member has an end portion acting as anchor element and extending outwardly from the surface of the body; **characterized in that** the angle between the longitudinal axis of the end portion of the anchor member and the rotation axis of the body is about 90 degree and that the angle between the longitudinal axis of the end portion of the anchor member and the surface of the body is between 0 and 45 degree.

2. An implantable cardiovascular device according to claim 1 wherein the cardiovascular device is a closure device being implantable into the left atrial appendage.

3. An implantable cardiovascular device according to claim 1 wherein the body is made of a biocompatible material such as a biocompatible metal or biocompatible plastic.

4. An implantable cardiovascular device according to claim 3 wherein the biocompatible metal is stainless steel, MP35N, gold, platinum, Pt/lr, Cu/Ni alloy like ISOTAN; or tungsten or a shape memory material, preferably nitinol.

5. An implantable cardiovascular device according to claim 1 wherein the body is a self-expandable mesh made of nitinol.

6. An implantable cardiovascular device according to claim 1 wherein the angle between the longitudinal axis of the end portion of the anchor member and the surface of the body is between 0 and 30 degree.

7. An implantable cardiovascular device according to claim 1 having 2 to 10 anchor members, preferably 4 to 8.

8. An implantable cardiovascular device according to claim 1, wherein the end portion of the anchor member is a straight wire, a curved wire, a sinus shaped wire, a tine shaped wire, an U-shaped wire, a V-shaped wire, a loop, a sling, a hook, a double hook, a litz wire, a barb, a T-bar, a screw.

9. An implantable cardiovascular device according to claim 1 wherein the anchor member is made of a biocompatible material such as a biocompatible metal or of biocompatible plastic.

10. An implantable cardiovascular device according to claim 9 wherein the anchor member is made of nitinol.

11. An implantable cardiovascular device according to claim 9 wherein the anchor member consists of a thin metallic wire having a thickness in the range of 0,05 mm to 0.5mm, preferably 0.05 to 0.3mm and a length in the range of 1mm to 3mm.
